# FASCICULE DE BREVET EUROPEEN

(11) **EP 1 651 564 B1**
(45) Date de publication et mention de la délivrance du brevet: **21.05.2008**
(21) Numéro de dépôt: 04767491.6
(22) Date de dépôt: 28.06.2004
(51) Int. Cl.: C01B 25/32

(54) **PLAQUETTES DE PHOSPHATE DE CALCIUM NANOMETRIQUE**
NANOSKALIGE CALCIUMPHOSPHATTABLETTEN
NANOSCALE CALCIUM PHOSPHATE TABLETS

(30) Priorité: 30.06.2003 FR 0307878
(43) Date de publication de la demande: 03.05.2006
(73) Titulaire: Innophos, Inc., Cranbury, NJ 08592 (US); CENTRE NATIONAL DE LA RECHERCHE SCIENTIFIQUE, 75794 Paris Cedex 16 (FR)
(72) Inventeur: CHANE-CHING, Jean-Yves, F-95600 EAUBONNE (FR); LEBUGLE, Albert, F-31650 SAINT ORENS DE GAMEVILLE (FR)
(74) Mandataire: OK pat AG
(86) Numéro de dépôt international: PCT/FR2004/001645
(87) Numéro de publication internationale: WO 2005/003027

(56) Documents cités:
- EP-A- 0 155 440
- WO-A-00/15194
- US-A- 5 073 357
- US-A- 5 427 754
- PATENT ABSTRACTS OF JAPAN vol. 2000, no. 08, 6 octobre 2000 (2000-10-06) & JP 2000 128513 A (NEW RAIMU KENKYUSHA:KK), 9 mai 2000 (2000-05-09)

## Description

La présente invention a pour objet des plaquettes de phosphate de calcium nanométrique, des dispersions de phosphate de calcium nanométrique et leurs procédés de préparation.

De nombreux industriels utilisent le phosphate de calcium sous différentes morphologies. Notamment, les morphologies les plus connues et utilisées sont les morphologies rhomboédriques, aiguilles ou larges plaquettes. Selon les structures de ce phosphate de calcium, les morphologies de phosphate de calcium seront différentes.

Le document EP-A-155440 décrit la préparation de plaquettes micrométriques de calcium de phosphate pour des compositions dentifrices.

En particulier, les plaquettes de phosphate de calcium sont utilisées comme charge renforçante, notamment pour renforcer les polymères ou une matrice polymérique. Cette application en renfort des plaquettes de phosphate de calcium permet d'améliorer les propriétés mécaniques des polymères ou de leur matrice.

Or, les technologies disponibles actuellement, ne permettent d'obtenir que des plaquettes de phosphate de calcium dont la taille est supérieure au micron, et qui sont sous forme d'agrégats, c'est à dire très peu individualisées.

Afin de répondre aux exigences des industriels il est devenu nécessaire de trouver des plaquettes de phosphate de calcium dont la taille est inférieure au micron tout en étant bien individualisées.

Aussi le problème que se propose de résoudre l'invention est de fournir des plaquettes de phosphates de calcium bien individualisées et dont la taille est de l'ordre de 250 nm à 800 nm.

Dans ce but l'invention propose des plaquettes de phosphate de calcium individualisées et dont la longueur est comprise entre 250 nm et 800 nm.

L'invention propose également des dispersions contenant les plaquettes selon l'invention ou des dispersions colloïdales obtenues par remise en suspension des dites plaquettes en présence d'un agent dispersant.

L'invention concerne aussi un procédé pour préparer des plaquettes selon l'invention.

Enfin l'invention a également pour objet l'utilisation des plaquettes pré-citées comme charge de renfort, agent de polissage, matériaux de construction, additif pour les formulations bucco-dentaires notamment les dentifrices ou agent d'encapsulation.

Les plaquettes de phosphate de calcium selon l'invention ont pour avantage de présenter des propriétés de barrière à la diffusion des gaz.

Les plaquettes de phosphate de calcium selon l'invention ont encore pour avantage d'être un bon matériau d'emballage, utilisable notamment dans le domaine alimentaire.

D'autres avantages et caractéristiques de la présente invention apparaîtront clairement à la lecture de la description et des exemples donnés à titre purement illustratif et non limitatif, qui vont suivre.

L'invention concerne tout d'abord des plaquettes de phosphate de calcium individualisées et dont la longueur est comprise entre 250 nm et 800 nm.

L'individualisation des plaquettes peut être montrée par analyse granulométrique basée sur un principe de sédimentation. On peut par exemple utiliser des appareils de mesure granulométrique tel que le Sedigraph, équipé d'un faisceau de rayons X pour analyser la sédimentation des plaquettes selon l'invention. La technique mise en oeuvre peut comprendre une première étape de dispersion en présence d'un agent dispersant, du type polyphosphate, et d'une étape de désagglomération par ultrasons, de puissance environ 600 watts, plus ou moins 20%, pendant 7 minutes. Il est également possible de faire directement la mesure sur une dispersion ou sur une dispersion colloïdale selon l'invention, sans étapes préalables.

Par plaquettes individualisées, on entend au sens de l'invention qu'au moins 80%, avantageusement au moins 90%, de préférence au moins 95%, en masse des plaquettes selon l'invention possèdent un diamètre équivalent inférieur ou égal à 200 nm.

Ce diamètre équivalent est avantageusement largement inférieur à la longueur des plaquettes visualisées par microscopie. Par diamètre équivalent, on entend la valeur déterminée par l'appareil d'analyse granulométrique basée sur un principe de sédimentation. Cette valeur est avantageusement calculée sur la base du diamètre d'une sphère virtuelle d'un matériau ayant la même vitesse de sédimentation que la vitesse de sédimentation des plaquettes selon l'invention.

Les plaquettes selon l'invention peuvent présenter 3 structures différentes : monétite ou monétite majoritaire ou apatite déficiente.

Tout d'abord les plaquettes selon l'invention peuvent présenter une structure monétite, bien cristalisée visible par diffraction aux rayons X. Ces plaquettes peuvent présenter un déplacement chimique compris entre -1,4 ppm et -1 ppm mesuré par RMN MAS du phosphore 31 qui peut être attribué à la structure monétite.

Dans certains cas, les plaquettes selon l'invention peuvent être constituées d'un mélange de plaquettes possédant plusieurs structures, en particulier un mélange de plaquettes à structure, monétite, brushite, apatite. Ce mélange est également appelé dans la suite de la description : plaquettes de phosphate de calcium à structure monétite majoritaire.

Dans ce mélange, certaines plaquettes peuvent présenter un déplacement chimique compris entre 3 ppm et 3,4 ppm mesuré par RMN MAS du phosphore 31 qui peut être attribué à une structure apatite.

Les plaquettes selon l'invention peuvent également présenter une structure apatite déficiente, bien cristalisée visible par diffraction aux rayons X.

Dans ce cas, les plaquettes de phosphate de calcium à structure apatite déficiente présentent avantageusement un rapport calcium sur phosphore compris entre 1,25 et 1,67, plus particulièrement compris entre 1,3 et 1,6. De plus le spectre RX de ces plaquettes de phosphate de calcium à structure apatite déficiente montre avantageusement des raies décalées vers les grandes distances par rapport à une structure hydroxy apatite.

La taille des plaquettes est visualisée de préférence par microscopie électronique à transmission (MET). Dans ce cas, Il est possible de faire l'analyse d'image à partir d'une dispersion, diluée ou non.

Les plaquettes selon l'invention ont avantageusement une longueur comprise entre 250 nm et 600 nm, de préférence comprise entre 250 nm et 400 nm.

Avantageusement 60% en nombre des plaquettes selon l'invention possèdent une taille inférieure ou égale à 500 nm, de préférence 70% et avantageusement 80%.

Les plaquettes selon l'invention ont avantageusement une épaisseur comprise 1 nm et 40 nm, de préférence entre 1 nm et 15 nm, plus particulièrement entre 2 nm et 6 nm.

Les plaquettes de phosphate de calcium selon l'invention présente avantageusement un rapport molaire calcium sur phosphore compris entre :
- 0,95 et 1,4 pour la structure monétite, de préférence compris entre 1,1 et 1,3 ;
- 0,95 et 1,4 pour la structure monétite mélangé à la structure brushite et apatite, de préférence compris entre 1,1 et 1,3 ;
- 1,25 et 1,67 pour la structure apatite déficiente, de préférence compris entre 1,3 et 1,6.

La structure monétite ou la structure apatitique déficiente peut être montrée par diffraction aux rayons X.

Les plaquettes de phosphate de calcium à structure monétite majoritaire présente un spectre aux rayons X montrant une apatite assez bien cristallisée, avec un paramètre c = 6,84A° inférieure au paramètre c des hydroxy apatites (c= 6,88 A°).

Les plaquettes de phosphate de calcium à structure monétite, ou à structure monétite majoritaire ou à structure apatite déficiente présentent avantageusement des surfaces spécifiques BET mesurées sur des produits séchés, comprises entre 40 et 100 m²/g, plus particulièrement entre 50 et 80 m²/g.

Les plaquettes de phosphate de calcium selon l'invention peuvent comprendre des éléments dopants.

De préférence ces éléments dopants sont choisis parmi les éléments alcalino-terreux comme le strontium, le magnésium, les éléments de terres rares comme l'yttrium ou les éléments de numéro atomique compris entre 57 et 71. D'autres éléments dopants peuvent également être envisagés, en fonction des différentes applications des dispersions selon l'invention.

L'invention concerne ensuite selon une première variante des dispersions colloïdales obtenues par remise en suspension de plaquettes de phosphate de calcium décrites ci-dessus en présence d'un agent dispersant.

L'invention concerne aussi selon une deuxième variante des dispersions contenant des plaquettes de phosphate de calcium décrites ci-dessus.

Dans le cas des deux variantes de dispersions selon l'invention au moins 80% des plaquettes en nombre ont une longueur comprise entre 250 nm et 600 nm, de préférence comprise entre 250 nm et 400 nm.

Les dispersions selon l'invention quelles que soient leurs variantes d'exécution peuvent également contenir au moins 50% de moles phosphore sous forme de structure monétite.

L'agent dispersant présent dans les dispersions colloïdales selon la première variante peut être choisi parmi les polyphosphates, en particulier les tripolyphosphates de sodium. Mais il est également possible de choisir tous agents dispersants couramment utilisés dans ce domaine et qui sont biens connus de l'homme du métier.

Les dispersions colloïdales selon la première variante présente avantageusement un rapport molaire Ra de moles de polyphosphate sur moles de calcium, Ra étant compris entre 0,02 et 0,2, de préférence compris entre 0,02 et 0,1.

Le polyphosphate est de préférence présent en surface des colloïdes ou dans la phase continue aqueuse.

L'invention a également pour objet un procédé de synthèse de plaquettes de phosphate de calcium selon l'invention.

Le procédé selon l'invention est mis en oeuvre de préférence par dissolution puis re-précipitation d'un précurseur approprié à base de brushite, ou de mélange brushite / apatite, et dans des conditions de dissolution re-précipitation définies ci-dessous.

Le procédé pour préparer les plaquettes de phosphate de calcium est caractérisé en ce qu'il comprend les étapes suivantes :
i) préparer une solution de sels de calcium dont le pH est compris entre 4 et 6;
ii) ajouter à la solution obtenue à l'étape i) une solution de phosphate, pendant une durée comprise entre 30 minutes et 4 heures et de manière à obtenir un rapport molaire calcium sur phosphore compris entre 1 et 2,5 et en maintenant constant le pH à une valeur comprise entre 4 et 6 ;
iii) traiter thermiquement la dispersion obtenue à l'étape ii) à une température comprise entre 50°C et 95°C ;
iv) séparer les plaquettes formées de la dispersion obtenue à l'étape iii) ; et en ce qu'il utilise dans au moins l'une des étapes i) ou ii) des solutions contenant un ion ammonium.

Selon un mode de réalisation particulier, les étapes i) et ii) peuvent être inversées. Dans ce cas la première étape du procédé est l'étape ii), et la deuxième étape est l'étape i).

Les plaquettes obtenues selon cette première variante du procédé présentent de préférence un déplacement chimique compris entre -1,4 ppm et -1 ppm mesuré par RMN MAS du phosphore 31 qui peut être attribué à la structure monétite.

Dans certains cas, les plaquettes obtenues selon cette première variante du procédé peuvent également présenter un déplacement chimique compris entre 3 ppm et 3,4 ppm mesuré par RMN MAS du phosphore 31 qui peut être attribué à la structure apatite. Dans ce cas précis, les plaquettes obtenues sont constituées d'un mélange de plaquettes possédant plusieurs structures, en particulier un mélange de plaquettes à structure, monétite, brushite, apatite. Il s'agit d'un mélange de plaquettes de phosphate de calcium à structure monétite majoritaire, comme indiqué plus haut.

Selon une autre variante, le procédé pour préparer les plaquettes de phosphate de calcium est caractérisé en ce qu'il comprend les étapes suivantes :
i) préparer une solution de sels de calcium dont le pH est compris entre 4 et 6;
ii) ajouter à la solution obtenue à l'étape i) une solution de phosphate pendant une durée comprise entre 30 minutes et 4 heures et de manière à obtenir un rapport molaire calcium sur phosphore compris entre 1 et 2,5 et en maintenant constant le pH à une valeur comprise entre 4 et 6 ;
iii) traiter thermiquement la dispersion obtenue à l'étape ii) à une température comprise entre 50°C et 95°C ;
iv) ajuster le pH de la dispersion obtenue à l'étape iii) a une valeur comprise entre 8 et 9,5 ;
v) séparer les plaquettes formées de la dispersion obtenue à l'étape iv) ;
et en ce qu'il utilise dans au moins l'une des étapes i) ou ii) des solutions contenant un ion ammonium.

Selon un mode de réalisation particulier, les étapes i) et ii) peuvent être inversées. Dans ce cas la première étape du procédé est l'étape ii), et la deuxième étape est l'étape i).

Les plaquettes obtenues selon cette deuxième variante du procédé présentent également de préférence une structure du type apatite déficiente, telle que définie plus haut.

Les indications suivantes sont valables quelle que soit la variante du procédé de l'invention mise en oeuvre.

L'étape ii) du procédé est de préférence réalisée par addition en continue et non pas de manière instantanée de la solution obtenue à l'étape i). Cette addition peut également se faire par un goutte à goutte ou une addition en discontinue à intervalle régulier de temps.

Cette addition de solution de phosphate à la solution de calcium est réalisée avec addition continue d'ions OH^{-,} de préférence de NH₄OH de manière à réguler le pH de la solution au pH de consigne. Le pH de consigne est de préférence compris entre 4 et 6.

La concentration en ions OH- de la solution servant à réguler le pH peut varier de préférence entre 1 M et 6M, plus particulièrement entre 2M et 4M.

L'addition d'ions OH⁻ à l'étape ii) peut être réalisée de manière à maintenir constant le pH de la dispersion régulée à pH compris entre 4 et 6 (pH de consigne), de préférence à pH égal à 5, ou à débit constant à l'aide d'une pompe. Par pH constant on entend un pH dont la valeur a été fixée à une valeur comprise entre 4 et 6 et dont le pH ne varie pas de plus 0,2 unités de pH par rapport à cette valeur.

La quantité d'ions OH⁻ versée est telle que le rapport molaire OH⁻ / P est compris entre 1 et 2,5, de préférence entre 1,5 et 2.

La solution de calcium utilisée selon le procédé de l'invention est avantageusement une solution de CaCl₂ ou de Ca(NO₃)₂. Cette solution peut éventuellement contenir des sels dopants comme ceux indiqués plus haut.

De préférence la concentration de la solution de calcium est comprise entre 1 M et 2,5M, de préférence entre 1,25M et 1,75M.

La solution de sel de phosphate utilisée selon le procédé de l'invention est avantageusement une solution de phosphate d'ammonium ou de sodium, notamment de (NH₄)₂(HPO₄) ou (NH₄)(H₂PO₄

Selon le procédé de l'invention, le rapport molaire calcium sur phosphore est avantageusement compris entre 1,3 et 1,7, plus particulièrement il est de 1,66.

A l'issu de l'étape ii), on obtient de préférence une dispersion sous forme d'un précipité. Par diffraction aux rayons X sur le précipité formé à l'issue de cette étape, qui a été centrifugé puis séché à 20°C, on observe une structure brushite CaHPO₄, 2H₂O Par microscopie, on observe une morphologie plaquettaire d'objet de taille micronique. Par résonance magnétique nucléaire du phosphore 31, on observe une structure brushite avec un déplacement chimique pouvant varier de δ ppm = 1,4 à δ ppm = 1,8, de préférence pouvant varier de 1,6ppm < δ < 1,8ppm.

Le procédé selon l'invention comprend une étape de traitement thermique, étape iii), dont la température est de préférence comprise entre 60°C et 90°C. Ce traitement thermique est aussi nommé mûrissement et a lieu pendant environ 3h à 24 h, de préférence pendant 3h à 16h. La montée en température peut avoir lieu en 5 minutes ou en 30 minutes.

L'étape iv) selon la première variante ou v) selon la deuxième variante du procédé selon l'invention, de séparation des plaquettes peut être réalisée par centrifugation, ou filtration. Ensuite les plaquettes sont de préférence séchées à température ambiante.

L'étape iv) selon la deuxième variante du procédé selon l'invention, peut être réalisée par addition d'une base à la dispersion obtenue à l'étape iii) de manière à obtenir une valeur de pH comprise entre 8 et 9,5. La montée en pH peut être réalisée par addition d'une base à la dispersion préalablement mise sous agitation à température ambiante. L'addition peut être instantanée ou effectuée de manière lente. Le temps d'addition peut être compris entre 1 minute et 24 heures de préférence entre 1 minute et 30 minutes. La dispersion est maintenue en pH pendant une durée pouvant varier de 5 minutes à 24 heures, de préférence entre 5 minutes et une heure.

Les dispersions colloïdales selon l'invention peuvent être réalisées entre autres selon le procédé décrit ci-après.

A l'issu de l'étape iv) selon la première variante ou v) selon la deuxième variante du procédé selon l'invention, le précipité solide obtenu peut être lavé en utilisant une solution aqueuse, de préférence de l'eau déminéralisée. Ce lavage est de préférence réalisé en utilisant 2 fois le volume du surnageant du précipité à laver. Puis le précipité lavé est séparé.

On redisperse le précipité lavé obtenu à l'aide d'une solution d'agent dispersant, en particulier à l'aide d'une solution de tripolyphosphate.

La concentration de la solution de tripolyphosphate est déterminée par le rapport molaire Rb de moles de polyphosphate sur mole de calcium, Rb étant compris entre 0,02 et 0,2, de préférence compris entre 0,02 et 0,15, et est également déterminée par la concentration finale en calcium de la dispersion.

Cette concentration finale en calcium est de préférence comprise entre 0,25M et 1,5M.

Après ajout de la solution d'agent dispersant, la solution est agitée pendant avantageusement 30 minutes à 6 heures.

Après ajout, la suspension peut être purifiée, par exemple par ultrafiltration sur une membrane de 3 KD par passage de 2 à 10 volumes d'eau.

On obtient une dispersion colloïdale et un culot.

Le culot est éliminé, par différentes techniques connues de l'homme du métier, notamment par filtration ou par centrifugation.

Enfin l'invention concerne l'utilisation des plaquettes de phosphate de calcium ou des dispersions selon l'invention comme charge de renfort, agent de polissage, matériaux de construction, additif pour les formulations bucco-dentaires notamment les dentifrices ou agent d'encapsulation.

Les exemples suivants illustrent l'invention sans toutefois en limiter la portée.

### EXEMPLES

### Exemple 1 : Procédé de préparation de plaquettes de phosphate de calcium à structure monétite

Etape i) : Une solution A est préparée par dissolution de 36,75 g de CaCl₂, 2H₂O (PM= 147 g/mole) dans 150 ml d'eau. On ajuste le pH à la valeur de 5 par addition de 0,3 ml d'une solution de HNO₃ à 0,01 M et on complète à 250 ml par de l'eau déminéralisée.
Etape ii) : Une solution B est préparée par dissolution de 19,8 g de (NH₄)₂HPO₄ (PM=132 g/mole) dans 200 ml d'eau. On neutralise cette solution à un pH de 5 par l'addition de 19 ml d'une solution de HNO₃ à 12M. Puis on complète à 250 ml par addition d'eau déminéralisée.
Dans un réacteur agité, à 20°C, on verse en fond de cuve la solution A de sel de calcium. On additionne en deux heures et à pH régulé la solution B de phosphate. La régulation de pH est obtenue à l'aide d'une solution de NH₄OH à 3M. La quantité d'ammoniaque 3M versée lors du maintien en pH est de 92 ml.
A la fin de l'addition on laisse sous agitation 30 minutes. Le rapport molaire est Ca/P= 1,66.
Etape iii) : La dispersion est ensuite portée à 80°C. La montée en température dure environ 30 minutes. Le temps de mûrissement à 80°C est de 16 heures.
Etape iv) : Après refroidissement de la dispersion, on recueille le produit solide par centrifugation. Le produit solide est lavé par 4 fois son volume d'eau. On sèche le produit à température ambiante.

### 1-1 Analyses RX et RMN d'un échantillon prélevé après l'étape de précipitation à 20°C (étape ii),

Les caractérisations ont été effectuées sur le produit lavé et séché à 20°C.
- On montre par diffraction aux rayons X principalement la présence de brushite bien cristallisée. Dans certains cas, se forme également de l'apatite minoritaire.
- Par RMN du phosphore 31, un décalage du déplacement chimique du pic attribuable à la brushite est observé pour le produit ( δ ppm = 1,73 ppm à comparer à δ ppm= 1,28 ppm pour la brushite classique).

### 1-2 Analyse d'un échantillon prélevé après mûrissement à 80°C (étape iii)

On montre l'individualisation des plaquettes par une analyse granulométrique du produit, réalisée avec un appareil de type Sédigraph. La mesure est basée sur un principe de sédimentation avec un détecteur de type RX sur une aliquote de 50 ml de la dispersion obtenue après l'étape iii). Après refroidissement de l'aliquote de la dispersion, on recueille le produit solide par centrifugation. Le produit solide est lavé par 4 fois son volume d'eau et revolumé à 50 ml. On additionne à la dispersion 0,77 g de sodium tripolyphosphate soit un rapport molaire tripolyphosphate / Calcium = 0,1 et on laisse sous agitation 30 minutes. La dispersion est mise sous ultrasons pendant 7 minutes. La cuve à ultrasons utilisée est équipée d'une sonde de diamètre 7 mn et de puissance maximale de 800W que l'on règle à 80%. L'analyse granulométrique du produit indique que 95 % des particules montrent un diamètre équivalent inférieur à 200 nm. Cette faible taille de diamètre équivalent confirme que les plaquettes observées par microscopie électronique à transmission sont bien individualisées.
Par microscopie (MET), on observe des plaquettes de dimensions environ 300 nm x 50 nm, étant entendu que 300 nm est la longueur et 50 nm est la largeur.

Les caractérisations suivantes ont été effectuées sur le produit lavé et séché à 20°C : par diffraction aux rayons X, on observe principalement la présence d'une structure monétite, avec un pic légèrement décalé vers les bas angles
On note également la présence de phase apatite en quantité minoritaire. Cette phase apatite peut être indexée sur une fiche correspondant à Ca_{9,54} P _{5,98} O _{23,58} CI _{1,60} (OH)_{2,74}.
Cette structure est déformée par rapport à la structure hydroxy apatite avec un paramètre a plus élevé et un paramètre c plus faible.

| | a | b | c | C |
|---|---|---|---|---|
| Ca₁₀ (PO₄)₆(OH)₂ (hydroxy apatite) | 9,432 | | 6,881 | 0,7295 |
| Ca_{9,54} P _{5,98} O _{23,58} Cl _{1,60} (OH)_{2,74} | 9,541 | | 6,838 | 0,7167 |

| | | | | |
|---|---|---|---|---|
| les valeurs a, b et c sont données en Angström, et C est le rapport c/a. | | | | |

Par diffraction des rayons X, et concernant la structure monétite, on montre également un pic de diffraction de très forte intensité correspondant à la direction 0h0, indiquant un plan des plaquettes perpendiculaire à la direction Oh0. La détermination de la taille des cristallites suivant cette direction montre la présence de domaines ordonnés de taille supérieure à 20 nm dans cette direction Oh0.
- Par RMN du phosphore 31, on montre la présence d'apatite de brushite et de monétite, en quantités respectives 35%, 10% et 55%. Néanmoins ces phases sont identifiées à des déplacements chimiques décalés par rapport aux déplacements chimiques classiquement attribués à ces phases.

| | δ ppm classique | δ ppm ( Produit préparé) |
|---|---|---|
| Apatite | + 2,9 ppm | +3,15 ppm |
| Brushite | + 1,28 ppm | |
| Monétite | -1,60 ppm | -1,15 ppm |

- Par infrarouge, on note la présence de monétite et d'apatite non stoechiométrique, mais néanmoins bien cristallisée.
- Par analyse chimique, le rapport Ca/P global est égal à environ Ca / Pₘₒₗₐᵢᵣₑ = 1,2

### Exemple 2 : Procédé de préparation de plaquettes de phosphate de calcium à structure apatite déficiente :

Les étapes i) ii) et iii) sont identiques aux étapes décrites dans l'exemple 1.
Etape iv) : sur une aliquote de 100 ml de la dispersion après mûrissement à 80°C (étape iii), refroidie à température ambiante et mise sous agitation, on additionne à l'aide d'une pompe 27 ml d'ammoniaque 1 M en 10 minutes. Le pH est pH 9. On laisse sous agitation pendant 5mn supplémentaires.
Etape v) : on centrifuge le produit. On lave le produit par de l'eau puis on sèche.

Après séchage à température ambiante, la diffraction aux rayons X montre une structure hydroxy apatite avec des raies décalées vers les grandes distances. Par microscopie électronique à transmission, on observe des plaquettes individualisées de taille environ 300 nm.

### Exemple 3 : Procédé de préparation de dispersions colloïdales de plaquettes de phosphate de calcium :

On reprend les conditions de l'exemple 1 jusqu'à l'étape iii). On prélève après refroidissement à température ambiante et mise sous agitation un volume de dispersion correspondant au tiers du volume total.
La dispersion est mise à centrifugée, on élimine le surnageant. On complète au volume initial par de l'eau déminéralisée et on met sous agitation. On répète une nouvelle fois l'opération de centrifugation, élimination du surnageant et addition d'eau au volume initial.
On additionne 3,06 g de tripolyphosphate de sodium PM= 368 g/mole soit un rapport molaire Rb= tripolyphosphate / Ca= 0,1.
On homogénéise par agitation pendant deux heures et on laisse reposer une nuit.
On récupère un surnageant colloïdal constituant la dispersion colloïdale selon l'invention.

## Revendications

1. Plaquette de phosphate de calcium individualisée présentant une structure monétite, ou monétite majoritaire ou apatite déficiente et dont la longueur est comprise entre 250 nm et 800 nm.

2. Plaquette de phosphate de calcium selon la revendication 1 **caractérisée en ce que** la longueur est comprise entre 250 nm et 600 nm, de préférence entre 250 nm et 400 nm.

3. Plaquette de phosphate de calcium selon l'une des revendications précédentes **caractérisée en ce que** son épaisseur est comprise entre 1 nm et 40 nm.

4. Plaquette de phosphate de calcium selon l'une des revendications 1 à 3 présentant un déplacement chimique compris entre -1,4 ppm et -1 ppm mesuré par RMN MAS du phosphore 31 attribué à la structure monétite.

5. Plaquette de phosphate de calcium selon l'une des revendications 1 à 3 présentant un déplacement chimique compris entre 3 ppm et 3,4 ppm mesuré par RMN MAS du phosphore 31 attribué à la structure apatite.

6. Plaquette de phosphate de calcium selon l'une des revendications 1 à 5 **caractérisée en ce qu'**elle présente un rapport molaire calcium sur phosphore compris entre 0,95 et 1,4 pour la structure monétite, de préférence compris entre 1,1 et 1,3 et compris entre 0,95 et 1,4 pour la structure monétite mélangé à la structure brushite et apatite, de préférence compris entre 1,1 et 1,3.

7. Plaquette de phosphate de calcium selon l'une des revendications 1 à 3 **caractérisée en ce qu'**elle présente un rapport molaire calcium sur phosphore compris entre 1,25 et 1,67 pour la structure apatite déficiente, de préférence compris entre 1,3 et 1,6.

8. Dispersion contenant des plaquettes de phosphate de calcium selon l'une des revendications 1 à 7.

9. Dispersion colloïdale obtenue par mise en suspension de plaquettes de phosphate de calcium selon l'une des revendications 1 à 7 en présence d'un agent dispersant.

10. Procédé pour préparer les plaquettes selon les revendications 1 à 6 **caractérisé en ce qu'**il comprend les étapes suivantes :
i) préparer une solution de sels de calcium dont le pH est compris entre 4 et 6;
ii) ajouter à la solution obtenue à l'étape i) une solution de phosphate, pendant une durée comprise entre 30 minutes et 4 heures et de manière à obtenir un rapport molaire calcium sur phosphore compris entre 1 et 2,5 et en maintenant constant le pH à une valeur comprise entre 4 et 6 ;
iii) traiter thermiquement la dispersion obtenue à l'étape ii) à une température comprise entre 50°C et 95°C ;
iv) séparer les plaquettes formées de la dispersion obtenue à l'étape iii) ;
et **en ce qu'**il utilise dans au moins l'une des étapes i) ou ii) des solutions contenant un ion ammonium.

11. Procédé pour préparer les plaquettes selon les revendications 1 à 3 et 7 **caractérisé en ce qu'**il comprend les étapes suivantes :
i) préparer une solution de sels de calcium dont le pH est compris entre 4 et 6;
ii) ajouter à la solution obtenue à l'étape i) une solution de phosphate pendant une durée comprise entre 30 minutes et 4 heures et de manière à obtenir un rapport molaire calcium sur phosphore compris entre 1 et 2,5 et en maintenant constant le pH à une valeur comprise entre 4 et 6 ;
iii) traiter thermiquement la dispersion obtenue à l'étape ii) à une température comprise entre 50°C et 95°C ;
iv) ajuster le pH de la dispersion obtenue à l'étape iii) a une valeur comprise entre 8 et 9,5 ;
v) séparer les plaquettes formées de la dispersion obtenue à l'étape iv) ;
et **en ce qu'**il utilise dans au moins l'une des étapes i) ou ii) des solutions contenant un ion ammonium.

12. Procédé selon les revendications 10 ou 11 **caractérisé en ce que** la solution de sels de calcium est une solution de CaCl₂ ou de Ca(NO₃)₂.

13. Procédé selon l'une des revendications 10 à 12 **caractérisé en ce que** la concentration de la solution de sels calcium est comprise entre 1 M et 2,5M, de préférence entre 1,25M et 1,75M.

14. Procédé selon l'une des revendications 10 à 13 **caractérisé en ce que** la solution de sel de phosphate est une solution de phosphate d'ammonium ou de sodium, notamment de (NH₄)₂(HPO₄) ou (NH₄)(H₂PO₄).

15. Procédé selon l'une des revendications 10 à 14 **caractérisé en ce que** le rapport molaire calcium sur phosphore est compris entre 1,3 et 1,7.

16. Procédé selon l'une des revendications 10 à 15 **caractérisé en ce que** le rapport molaire calcium sur phosphore est de 1,66.

17. Procédé selon l'une des revendications 10 à 16 **caractérisé en ce que** la température du traitement thermique à l'étape iii) est comprise entre 60°C et 90°C.

18. Utilisation des plaquettes selon l'une des revendications 1 à 7 ou d'une dispersion selon les revendications 8 ou 9 comme charge de renfort, agent de polissage, matériaux de construction, additif pour les formulations bucco-dentaires notamment les dentifrices ou agent d'encapsulation.

## Claims

1. Individualised calcium phosphate platelet which exhibits a monetite, predominant monetite or deficient apatite structure and the length of which is between 250 nm and 800 nm.

2. Calcium phosphate platelet according to claim 1, **characterised in that** the length is between 250 nm and 600 nm, preferably between 250 nm and 400 nm.

3. Calcium phosphate platelet according to any of the preceding claims, **characterised in that** its thickness is between 1 nm and 40 nm.

4. Calcium phosphate platelet according to any one of claims 1 to 3, exhibiting a chemical shift of between -1.4 ppm and -1 ppm as measured by phosphorus-31 MAS NMR attributed to the monetite structure.

5. Calcium phosphate platelet according to any one of claims 1 to 3, exhibiting a chemical shift of between 3 ppm and 3.4 ppm, measured by phosphorus-31 MAS NMR attributed to the apatite structure.

6. Calcium phosphate platelet according to any one of claims 1 to 5, **characterised in that** it has a calcium to phosphorus molar ratio of between 0.95 and 1.4 for the monetite structure, preferably between 1.1 and 1.3, and between 0.95 and 1.4 for the monetite structure mixed with the brushite and apatite structure, preferably between 1.1 and 1.3.

7. Calcium phosphate platelet according to any one of claims 1 to 3, **characterised in that** it has a calcium to phosphorus molar ratio of between 1.25 and 1.67 for the deficient apatite structure, preferably between 1.3 and 1.6.

8. Dispersion containing calcium phosphate platelets according to any one of claims 1 to 7.

9. Colloidal dispersion obtained by suspending calcium phosphate platelets according to any one of claims 1 to 7 in the presence of a dispersing agent.

10. Process for preparing the platelets according to claims 1 to 6, **characterised in that** it includes the following steps:
i) preparing a solution of calcium salts, the pH of which is between 4 and 6;
ii) adding a phosphate solution to the solution obtained in step i) over a period of time of between 30 minutes and 4 hours, so as to obtain a calcium to phosphorus molar ratio of between 1 and 2.5 and maintaining the pH constant at a value of between 4 and 6;
iii) heat treating the dispersion obtained in step ii) at a temperature of between 50°C and 95°C;
iv) separating the platelets formed from the dispersion obtained in step iii);
and **in that** the process uses in at least one of steps i) or ii) solutions containing an ammonium ion.

11. Process for preparing the platelets according to claims 1 to 3 and 7, **characterised in that** it includes the following steps:
i) preparing a solution of calcium salts, the pH of which is between 4 and 6;
ii) adding a phosphate solution to the solution obtained in step i) over a period of time of between 30 minutes and 4 hours, so as to obtain a calcium to phosphorus molar ratio of between 1 and 2.5 and maintaining the pH constant at a value of between 4 and 6;
iii) heat treating the dispersion obtained in step ii) at a temperature of between 50°C and 95°C;
iv) adjusting the pH of the dispersion obtained in step iii) to a value of between 8 and 9.5;
v) separating the platelets formed from the dispersion obtained in step iv);
and **in that** the process uses in at least one of steps i) or ii) solutions containing an ammonium ion.

12. Process according to either claim 10 or claim 11, **characterised in that** the solution of calcium salts is a CaCl₂ or Ca(NO₃)₂ solution.

13. Process according to any one of claims 10 to 12, **characterised in that** the concentration of calcium salts in the solution is between 1 M and 2.5 M, preferably between 1.25 M and 1.75 M.

14. Process according to any one of claims 10 to 13, **characterised in that** the phosphate salt solution is an ammonium or sodium phosphate solution, in particular (NH₄)₂(HPO₄) or (NH₄)(H₂PO₄).

15. Process according to any one of claims 10 to 14, **characterised in that** the calcium to phosphorous molar ratio is between 1.3 and 1.7.

16. Process according to any one of claims 10 to 15, **characterised in that** the calcium to phosphorus molar ratio is 1.66.

17. Process according to any one of claims 10 to 16, **characterised in that** the temperature of the heat treatment in step iii) is between 60°C and 90°C.

18. Use of the platelets according to any one of claims 1 to 7 or of a dispersion according to either claim 8 or claim 9 as a reinforcing filler, polishing agent, building materials, additive for oral formulations, in particular dentifrices, or encapsulating agent.

## Patentansprüche

1. Individualisiertes Kalziumphosphatplättchen mit einer Monetit-, oder einer hauptsächlichen Monetit- oder einer defizienten Apatitstruktur und mit einer Länge zwischen 250 nm und 800 nm.

2. Kalziumphosphatplättchen nach Anspruch 1, **dadurch gekennzeichnet, dass** die Länge zwischen 250 und 600 nm beträgt, vorzugsweise zwischen 250 und 400 nm.

3. Kalziumphosphatplättchen nach einem der vorausgehenden Ansprüche, **dadurch gekennzeichnet, dass** ihre Dicke zwischen 1 nm und 40 nm beträgt.

4. Kalziumphosphatplättchen nach einem der Ansprüche 1 bis 3 mit einer chemischen Verschiebung zwischen -1.4 ppm und -1 ppm, die durch Phosphor-31-NMR-Spektroskopie gemessen und der Monetitstruktur zugeschrieben wird.

5. Kalziumphosphatplättchen nach einem der Ansprüche 1 bis 3 mit einer chemischen Verschiebung zwischen 3 ppm und 3.4 ppm, die durch Phosphor-31-NMR-Spektroskopie gemessen und der Apatitstruktur zugeschrieben wird.

6. Kalziumphosphatplättchen nach einem der Ansprüche 1 bis 5, **dadurch gekennzeichnet, dass** sie ein Molverhältnis Kalzium/Phosphor zwischen 0.95 und 1.4 hinsichtlich der Monetitstruktur, vorzugsweise zwischen 1.1 und 1.3, und hinsichtlich der Monetitstruktur vermischt mit der Brushit- und Apatitstruktur ein Molverhältnis zwischen 0.95 und 1.4, vorzugsweise zwischen 1.1 und 1.3 aufweist.

7. Kalziumphosphatplättchen nach einem der Ansprüche 1 bis 3, **dadurch gekennzeichnet, dass** sie ein Molverhältnis Kalzium/Phosphor zwischen 1.25 und 1.67 hinsichtlich der defizienten Apatitstruktur, vorzugsweise zwischen 1.3 und 1.6 aufweist.

8. Dispersion mit Kalziumphosphatplättchen nach einem der Ansprüche 1 bis 7.

9. Kolloidale Dispersion, die durch Suspension von Kalziumphosphatplättchen nach einem der Ansprüche 1 bis 7 mit einem Dispergiermittel erhalten wird.

10. Präparationsverfahren für die Plättchen nach den Ansprüchen 1 bis 6, **dadurch gekennzeichnet, dass** es folgende Schritte umfasst:
i) Präparieren einer Kalziumsalzlösung mit einem pH-Wert zwischen 4 und 6;
ii) Hinzufügen einer Phosphatlösung zur in Schritt i) erhaltenen Lösung während einer Dauer zwischen 30 Minuten und 4 Stunden, sodass ein Molverhältnis Kalzium/Phosphor zwischen 1 und 2.5 erzielt und der pH-Wert konstant auf einem Wert zwischen 4 und 6 gehalten wird;
iii) Wärmebehandlung der in Schritt ii) erhaltenen Dispersion bei einer Temperatur zwischen 50° und 95°C;
iv) Abtrennen der Plättchen, die aus der in Schritt iii) erhaltenen Dispersion gebildet werden;
und **dadurch gekennzeichnet, dass** mindestens in einem der Schritte i) oder ii) Lösungen verwendet werden, die ein Ammoniumion enthalten.

11. Präparationsverfahren für die Plättchen nach den Ansprüchen 1 bis 3 und 7, **dadurch gekennzeichnet, dass** es folgende Schritte umfasst:
i) Präparieren einer Kalziumsalzlösung mit einem pH-Wert zwischen 4 und 6;
ii) Hinzufügen einer Phosphatlösung zur in Schritt i) erhaltenen Lösung während einer Dauer zwischen 30 Minuten und 4 Stunden, sodass ein Molverhältnis Kalzium/Phosphor zwischen 1 und 2.5 erzielt und der pH-Wert konstant auf einem Wert zwischen 4 und 6 gehalten wird;
iii) Wärmebehandlung der in Schritt ii) erhaltenen Dispersion bei einer Temperatur zwischen 50° und 95°C;
iv) Justieren des pH-Werts der in Schritt iii) erhaltenen Dispersion auf einen Wert zwischen 8 und 9.5;
v) Abtrennen der Plättchen, die aus der in Schritt iv) erhaltenen Dispersion gebildet werden;
und **dadurch gekennzeichnet, dass** mindestens in einem der Schritte i) oder ii) Lösungen verwendet werden, die ein Ammoniumion enthalten.

12. Verfahren nach den Ansprüchen 10 oder 11, **dadurch gekennzeichnet, dass** die Kalziumsalzlösung eine CaCl₂- oder Ca(NO₃)₂-Lösung ist.

13. Verfahren nach einem der Ansprüche 10 bis 12, **dadurch gekennzeichnet, dass** die Konzentration der Kalziumsalzlösung zwischen 1M und 2.5M, vorzugsweise zwischen 1.25M und 1.75M liegt.

14. Verfahren nach einem der Ansprüche 10 bis 13, **dadurch gekennzeichnet, dass** die Phosphatsalzlösung eine Ammonium- oder Natriumphosphatlösung, insbesondere eine (NH₄)₂(HPO₄)- oder (NH₄)(H₂PO₄)-Lösung, ist.

15. Verfahren nach einem der Ansprüche 10 bis 14, **dadurch gekennzeichnet, dass** das Molverhältnis Kalzium/Phosphor zwischen 1.3 und 1.7 liegt.

16. Verfahren nach einem der Ansprüche 10 bis 15, **dadurch gekennzeichnet, dass** das Molverhältnis Kalzium/Phosphor 1.66 beträgt.

17. Verfahren nach einem der Ansprüche 10 bis 16, **dadurch gekennzeichnet, dass** die Temperatur der Wärmebehandlung in Schritt iii) zwischen 60° und 90°C beträgt.

18. Anwendung der Plättchen nach einem der Ansprüche 1 bis 7 oder einer Dispersion nach den Ansprüchen 8 oder 9 als Verstärkungsfüller, Poliermittel, Baustoff, Zusatz für Mund- und Zahnreinigungsmittel, insbesondere Zahnpasten oder Verkapselungsmittel.
